# EUROPEAN PATENT APPLICATION

(11) **EP 3 489 670 A1**
(43) Date of publication of application: **29.05.2019**
(21) Application number: 17830998.5
(22) Date of filing: 18.07.2017
(51) Int. Cl.: G01N 27/12

(54) **GAS SENSOR**

(30) Priority: 19.07.2016 JP 2016141501
(71) Applicant: NGK Spark Plug Co., Ltd., Nagoya-shi, Aichi 467-8525 (JP)
(72) Inventor: UEYAMA, Takeshi, Nagoya-shi, Aichi 467-8525 (JP); YOKOYAMA, Takahiro, Nagoya-shi, Aichi 467-8525 (JP); ITO, Hiromasa, Nagoya-shi, Aichi 467-8525 (JP); NAKAGAWA, Shinichi, Nagoya-shi, Aichi 467-8525 (JP); AOYAMA, Shigeya, Nagoya-shi, Aichi 467-8525 (JP); MORITA, Kazuto, Nagoya-shi, Aichi 467-8525 (JP)
(74) Representative: J A Kemp
(86) International application number: PCT/JP2017/025956
(87) International publication number: WO 2018/016482

(57) **Abstract**

[Problem] To provide a gas sensor in which a sensor element is fixedly suspended within an opening of a wiring board, whose damage due to thermal stress is suppressed, and whose electricity conducting members are less likely to deform even when the gas sensor falls.

[Means for Solution] A gas sensor 1 includes a wiring board 50 having an opening 50h, a sensor element 40 disposed within the opening of the wiring board and having a detection section 43 and a heater section 42, and a plurality of electricity conducting members 61, 63 each of which has a first end portion joined to the wiring board and a second end portion joined to the sensor element and which establish electrical connection between the wiring board and the sensor element. The plurality of electricity conducting members fixedly suspend the sensor element within the opening of the wiring board. At least one of the electricity conducting members is a particular electricity conducting member 61 having a plate-like shape, and the particular electricity conducting member includes at least one bent portion 61w between the second end portion 61a2 joined to the sensor element and the first end portion 61a1 joined to the wiring board, the bent portion 61w being bent in a thickness direction T of the particular electricity conducting member.

## Description

### TECHNICAL FIELD

The present invention relates to a gas sensor that detects the concentration of a particular gas.

### BACKGROUND ART

One known metal oxide sensor has a structure in which a sensor element is fixedly suspended within an opening of a wiring board by, for example, bonding wires (Patent Document 1) .

By fixing the sensor element in a suspended manner, the thermal capacity of the sensor element and the thermal influence from the surroundings can be reduced, and the accuracy of gas detection and the responsiveness of the sensor element can thereby be improved.

Another known gas sensor includes a plurality of pins fixed to a base and a plurality of leads formed from a wire and used to connect a sensor element to the plurality of pins and has a structure in which the sensor element is fixedly suspended through the leads (Patent Document 2).

### PRIOR ART DOCUMENTS

### PATENT DOCUMENTS

Patent Document 1: Japanese Patent Application Laid-Open (*kokai*) No. 2007-298508 (FIG. 1)
Patent Document 2: Japanese Patent Application Laid-Open (*kokai*) No. 2006-208358 (FIGS. 1 and 2)

### SUMMARY OF THE INVENTION

### PROBLEMS TO BE SOLVED BY THE INVENTION

Incidentally, such a sensor element includes not only a detection section for detecting the concentration of a particular gas, but also a heater for heating the detection section to an operation temperature. Therefor, the sensor of Patent Document 1 has a problem in that, due to thermal stress attributable to repetition of a phenomenon in which the bonding wires are heated as a result of heating by the heater and the bonding wires are then cooled (specifically, metal fatigue caused by accumulation of the thermal stress), the bonding wires contract and break, or energization pads of the sensor element joined to the bonding wires are pulled so that the energization pads come off. Also, in the sensor of Patent Document 2, each lead formed from a wire has periodic bent portions along the width direction of the sensor element. Therefore, when the above-mentioned thermal stress is produced by the heater disposed on the sensor element, the leads can absorb the thermal contraction. However, the leads are formed from a wire and the bent portions bend along the width direction of the sensor element. Therefore, when the sensor falls and receives an impact, the leads deform easily. Due to excessive deformation of the leads, the position of the sensor element may change from the initial position, which may lower the accuracy of gas detection.

Accordingly, it is an object of the present invention to provide a gas sensor in which a sensor element is fixedly suspended within an opening of a wiring board, whose damage due to thermal stress is suppressed, and whose electricity conducting members are less likely to deform even when the gas sensor falls.

### MEANS FOR SOLVING THE PROBLEMS

In order to solve the above-described problems, the present invention provides a gas sensor comprising: a wiring board having an opening; a sensor element disposed within the opening of the wiring board and including a detection section for detecting the concentration of a particular gas and a heater section for heating the detection section; and a plurality of electricity conducting members each of which has a first end portion joined to the wiring board and a second end portion joined to the sensor element and which establish electrical connection between the wiring board and the sensor element, the plurality of electricity conducting members fixedly suspending the sensor element within the opening of the wiring board, wherein at least one of the electricity conducting members is a particular electricity conducting member having a plate-like shape, and the particular electricity conducting member includes at least one bent portion between the second end portion joined to the sensor element and the first end portion joined to the wiring board, the bent portion being bent in a thickness direction of the particular electricity conducting member.

In this gas sensor, even when the particular electricity conducting member is heated as a result of the heating by the heater section and is then cooled; i.e., when the particular electricity conducting member receives a thermal stress, its bent portion absorbs thermal contraction of the particular electricity conducting member. As a result, it is possible to prevent breakage of the particular electricity conducting member and prevent a connection portion of the sensor element or the wiring board joined to the particular electricity conducting member from being pulled and coming off. Thus, damage of the gas sensor can be prevented. In particular, since the particular electricity conducting member has a plate-like shape, as compared with a wire, the particular electricity conducting member is higher in rigidity and can secure a wider joining region for joining to the wiring board, thereby increasing the strength of joining between the particular electricity conducting member and the wiring board. Therefore, even when the particular electricity conducting member receives a thermal stress, damage of the gas sensor can be suppressed well.

Also, even when the gas sensor falls and receives an impact, deformation of the particular electricity conducting member can be suppressed, because the particular electricity conducting member has a plate-like shape and is therefore high in rigidity, and the particular electricity conducting member has the bent portion bent in the thickness direction and can effectively relieve the impact force generated as a result of the falling. Therefore, even when the gas sensor receives an impact as a result of falling, a deterioration in the gas detection accuracy is suppressed. Specially, even when the gas sensor receives an impact as a result of falling, a positional shift of the sensor element suspended at a particular position within the opening of the wiring board is less likely to occur, and the volume of the gas atmospheric space around the sensor element (the detection section) is restrained from changing as a result of falling of the gas sensor. The positional shift of the sensor element and the changing of the gas atmospheric space would otherwise occur as a result of deformation of the particular electricity conducting member. Thus, a deterioration in the gas detection accuracy is suppressed.

Notably, in the case where the particular electricity conducting member has the shape of a plate whose width is two times or more a thickness thereof, the particular electricity conducting member can have increased rigidity, thereby effectively exhibiting the effect of absorbing the thermal contraction of the particular electricity conducting member and the effect of relieving the impact force generated as a result of falling of the gas sensor. The width of the particular electricity conducting member is preferably three times or more the thickness. Although no particular limitation is imposed on the upper limit of the width, from the viewpoint of miniaturization of the gas sensor, the width of the particular electricity conducting member is preferably not greater than twelve times the thickness of the particular electricity conducting member.

In the gas sensor of the present invention, the bent portion may be bent into an arch shape.

In this gas sensor, since the bent portion can extend and contract smoothly, thermal contraction of the particular electricity conducting member can be absorbed more stably.

In the gas sensor of the present invention, preferably, the particular electricity conducting member has, as a single bent portion, the bent portion bent unidirectionally in the thickness direction of the particular electricity conducting member, and the bent portion has a height two times or more the thickness of the particular electricity conducting member.

In this case, since the particular electricity conducting member having a single bent portion is simple in structure, a gas sensor which is inexpensive and compact can be provided. Also, in the case where the height of the bent portion bent unidirectionally in the thickness direction is rendered two times or more the thickness (preferably, three times or more the thickness), the thermal contraction of the particular electricity conducting member can be absorbed without fail, and the impact force generated as a result of falling of the gas sensor can be relieved without fail. Although no particular limitation is imposed on the upper limit of the height of the bent portion, from the viewpoint of miniaturization of the gas sensor, the height of the bent portion is preferably not greater than six times the thickness of the particular electricity conducting member.

The gas sensor of the present invention may be configured such that the number of the particular electricity conducting members is at least two, the sensor element has a quadrilateral shape in plan view, one of the particular electricity conducting members is joined to the sensor element and the wiring board in such a manner as to extend across a first side of the sensor element which is one of four sides of the sensor element defining a peripheral edge thereof, and the other of the particular electricity conducting members is joined to the sensor element and the wiring board in such a manner as to extend across a second side of the sensor element which is another of the four sides and is located opposite the first side.

In this gas sensor, since the particular electricity conducting members fixedly suspend the first side and the second side (the opposite sides) of the sensor element, the sensor element can be fixed more reliably. In particular, in the case where the particular electricity conducting members fixedly suspend the sensor element at positions near the four corners of the sensor element, the sensor element can be fixed more firmly.

In the gas sensor of the present invention, an electrically conductive energization pad which is welded to the particular electricity conducting member may be provided on a surface of the sensor element, and a main component of a material used to form the energization pad may be the same as a main component of a material used to form the particular electricity conducting member.

In this gas sensor, since adhesion between the particular electricity conducting member and the energization pad is improved, the sensor element can be fixed more reliably.

### EFFECTS OF THE INVENTION

According to the present invention, there can be obtained a gas sensor in which a sensor element is fixedly suspended within an opening of a wiring board, whose damage due to thermal stress is suppressed, and whose electricity conducting members are less likely to deform even when the gas sensor falls.

### BRIEF DESCRIPTION OF THE DRAWINGS

[FIG. 1] Exploded perspective view of a gas sensor according to an embodiment of the present invention.
[FIG. 2] Top view of the gas sensor according to the embodiment of the present invention.
[FIG. 3] Bottom view of the gas sensor according to the embodiment of the present invention.
[FIG. 4] Perspective view showing a particular electricity conducting member.
[FIG. 5] Perspective view of the particular electricity conducting member extracted from FIG. 4.

### MODES FOR CARRYING OUT THE INVENTION

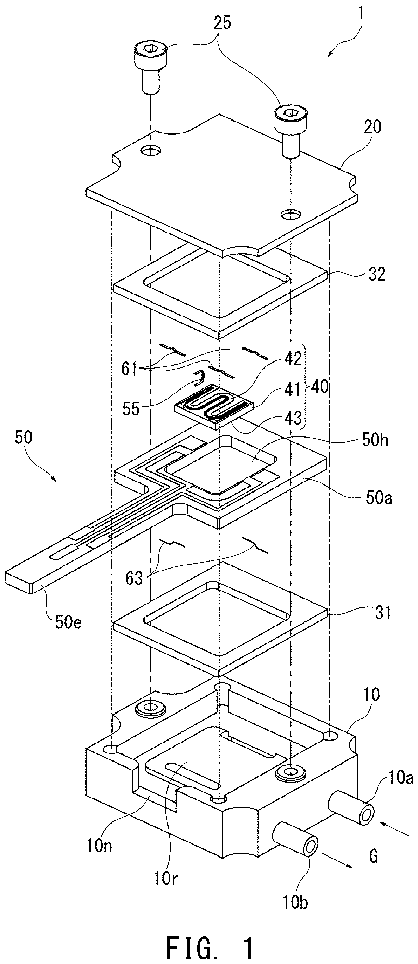

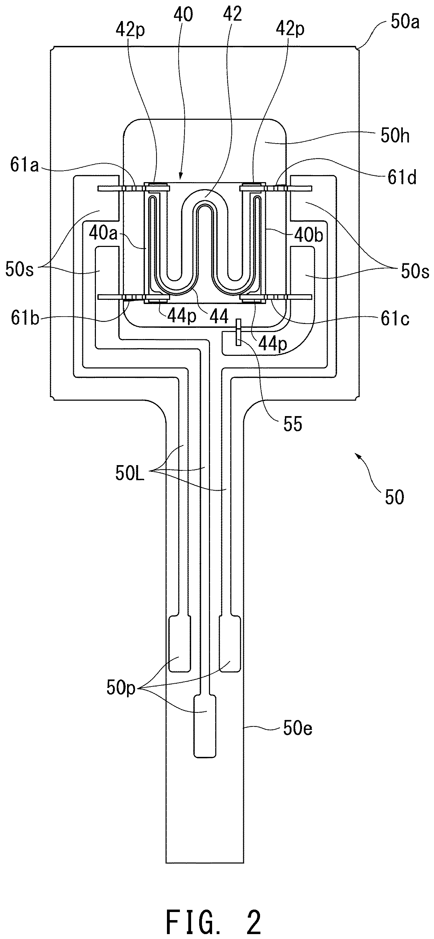

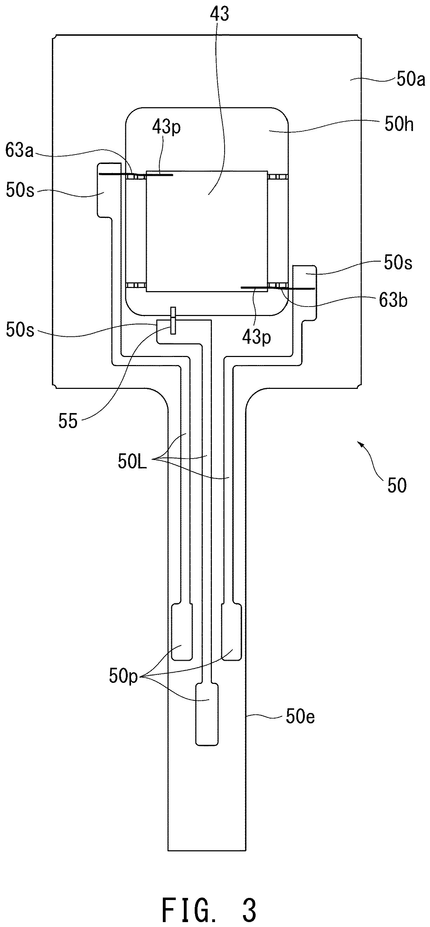

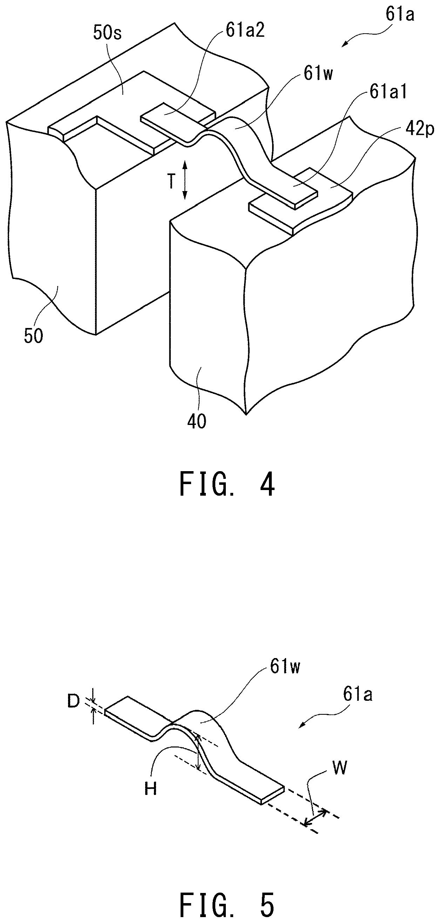

The present invention will next be described in detail with reference to the drawings. FIG. 1 is an exploded perspective view of a gas sensor 1 of an embodiment of the present invention, and FIGS. 2 and 3 are top and bottom views, respectively, of the gas sensor 1. FIG. 4 is a perspective view showing a particular electricity conducting member 61.

In FIG. 1, the gas sensor 1 includes: a case 10 having a generally rectangular box shape with an opening on its upper surface (an upward surface in FIG. 1); a lid 20 that covers the opening of the case 10; a ceramic wiring board (hereinafter simply referred to as "wiring board") 50 accommodated in the case 10; rectangular frame-shaped seal members (gaskets) 31 and 32; a sensor element 40 disposed within an opening 50h of the ceramic wiring board 50; and electricity conducting members 61 and 63 which fixedly suspend the sensor element 40 within the opening 50h. Notably, although the number of the electricity conducting members 61 is four, in FIG. 1, one of the electricity conducting members 61 is not illustrated because the one electricity conducting member 61 is disposed under the seal member 31. Also, the number of the electricity conducting members 63 is two.

The case 10 has an accommodation space 10r, a rectangular notch 10n extending downward from the upper end of the case 10, and pipe-shaped introduction and discharge tubes 10a and 10b used as connection ports for pipes. The introduction tube 10a and the discharge tube 10b protrude rightward from one side surface (the right side-surface in FIG. 1) of the case 10, and the bores of the tubes 10a and 10b are in communication with the accommodation space 10r.

The wiring board 50 includes a rectangular frame-shaped distal end portion 50a and a proximal end portion 50e that has a narrower width than the distal end portion 50a and extends outward (leftward in FIG. 1) from one side of the distal end portion 50a.

Each of the seal members 31 and 32 and the distal end portion 50a of the ceramic wiring board 50 is sized so as to be exactly fittable into the accommodation space 10r of the case 10. The seal member 31, the distal end portion 50a, and the seal member 32 stacked in this order from the case 10 side are accommodated in the accommodation space 10r. The proximal end portion 50e of the ceramic wiring board 50 protrudes from the notch 10n to the outside of the case 10.

Then the lid 20 is placed on the seal member 32 and fastened to the case 10 with bolts 25. The seal members 31 and 32 are thereby pressed between the case 10 and the lid 20, and the gap between the case 10 and the ceramic wiring board 50 is hermetically sealed.

As a result, a particular gas G introduced from the introduction tube 10a into the accommodation space 10r comes into contact with the sensor element 40, and the concentration of the particular gas is detected. Then the gas is discharged to the outside through the discharge tube 10b.

Notably, the sensor element 40 has a generally rectangular plate-like shape. A heater section 42 is disposed on the upper surface side (the upward side in FIG. 1) of a base 41, and a detection section 43 is disposed on the lower surface side of the base 41. In this structure, the detection section 43 and the heater section 42 are stacked on the upper and lower sides, respectively, of the base 41 and integrated together.

The electrical properties of the detection section 43 change in proportion to the concentration of the particular gas component, and the concentration of the particular gas component is detected by detecting an electric signal corresponding to the changing electrical properties. When the heater section 42 is energized, the heater section 42 generates heat, thereby heating the detection section 43 to its operating temperature. Output terminals of the detection section 43 and energization terminals of the heater section 42 are fixed and electrically connected to the wiring board 50 through the electricity conducting members 61 and 63 in a suspended manner.

The base 41 may be, for example, a ceramic substrate. The detection section 43 may be formed of, for example, an oxide semiconductor. The heater section 42 may be, for example, a circuit formed on a surface of the base 41 and serving as a heat-generating resistor.

A plurality of electrically conductive pads 50p electrically connected to the detection section 43 and the heater section 42 through the electricity conducting members 61 and 63 and lead traces 50L are disposed on the front and back surfaces of the proximal end portion 50e of the wiring board 50. The electric signal outputted from the detection section 24 is outputted to the outside through the electrically conductive pads 50p of the wiring board 50, and the heater section 42 is energized by electric power supplied from the outside through the electrically conductive pads 50p and thereby generates heat.

As shown in FIG. 2, the sensor element 40 is formed into a rectangular shape in plan view, and two electricity conducting members 61a and 61b extending across a first side 40a (the left side in FIG. 2) of the sensor element 40 are joined to the wiring board 50 and to opposite side edges of the sensor element 40. Similarly, other two electricity conducting members 61c and 61d extending across a second side 40b (the right side in FIG. 2) opposed to the first side 40a are joined to the wiring board 50 and to the opposite side edges of the sensor element 40.

These four electricity conducting members 61a to 61d are bent as shown in FIG. 4, which will be described in detail later, and correspond to the "particular electricity conducting member" in the claims.

A plurality of (four in FIG. 2) element peripheral pads 50s are formed on the front surface of the wiring board 50 so as to surround the opening 50h. These element peripheral pads 50s are connected to the respective electrically conductive pads 50p (three pads in FIG. 3) through the lead traces 50L.

Energization pads 42p are formed at opposite ends of the heat-generating resistor forming the heater section 42. The energization pads 42p are disposed to face the first side 40a and the second side 40b, respectively. Two element peripheral pads 50s are disposed so as to be opposed to the respective energization pads 42p. The electricity conducting members 61a and 61d establish connection between the energization pads 42p and the respective element peripheral pads 50s opposed to the energization pads 42p.

A temperature sensor 44 is disposed along the pattern of the heater section 42, and energization pads 44p are formed at opposite ends of the temperature sensor 44. The energization pads 44p are also disposed to face the first side 40a and the second side 40b, respectively. Two element peripheral pads 50s are disposed so as to be opposed to the respective energization pads 44p, and the electricity conducting members 61b and 61c establish connection between the energization pads 44p and the respective element peripheral pads 50s opposed to the energization pads 44p.

Notably, the electricity conducting members 61a and 61d are disposed on the upper side of the sensor element 40 in FIG. 2, and the electricity conducting members 61b and 61c are disposed on the lower side of the sensor element 40. As a result, the four electricity conducting members 61a to 61d fixedly suspend the upper and lower ends of the first side 40a and the second side 40b; namely, fixedly suspend the sensor element 40 at positions near the four corners thereof.

Also, a U-shaped conductive member 55 is connected to the lower-right element peripheral pad 50s in FIG. 2. This conductive member 55 is connected to an element peripheral pad 50s on the opposite surface of the wiring board 50 (see FIG. 3), and serves as the ground.

As shown in FIG. 3, on the opposite surface of the sensor element 40 as well, a plurality of (three in FIG. 3) element peripheral pads 50s are formed on the back surface of the ceramic wiring board 50 so as to surround the opening 50h. These element peripheral pads 50s are connected to the respective electrically conductive pads 50p (three pads in FIG. 3) through the lead traces 50L.

Two electricity conducting members 63a and 63b are joined to two energization pads 43p connected to the detection section 43 of the sensor element 40 and are joined to the element peripheral pads 50s of the wiring board 50 opposed to these energization pads 43p. Since the electricity conducting members 63a and 63b are not bent, the electricity conducting members 63a and 63b do not correspond to the "particular electricity conducting member" in the claims.

The electricity conducting members 61 and the conductive member 55 may be formed of, for example, Pt, and the electricity conducting members 63 may be formed of, for example, Au. They can be electrically connected to the respective energization pads and element peripheral pads by, for example, welding. Notably, in the present embodiment, the electricity conducting members 61 are joined to the corresponding energization pad and the corresponding element peripheral pad by means of electric resistance welding. The energization pads can be formed by applying, for example, a Pt paste to the base 41 of the sensor element 40 by printing and then firing the printed Pt paste. The element peripheral pads 50s, the lead traces 50L, and the electrically conductive pads 50p can be formed by printing, for example, an Au paste onto the wiring board 50 and then firing the printed Au paste.

Next, the particular electricity conducting member 61a will be described with reference to FIG. 4. FIG. 4 is a perspective view of the particular electricity conducting member 61a located at the upper left corner of the sensor element 40 in FIG. 2. FIG. 5 is a perspective view of the particular electricity conducting member 61a extracted from FIG. 4.

The particular electricity conducting member 61a is not a wire but is a plate. The particular electricity conducting member 61a has a single bent portion 61w between a first end portion 61a1 joined to the energization pad 42p of the sensor element 40 and a second end portion 61a2 joined to the element peripheral pad 50s of the wiring board 50. The bent portion 61w bends unidirectionally in the thickness direction T thereof (namely, without meandering bidirectionally in the thickness direction T) to thereby form an arch-like shape. The width W of the plate-shaped particular electricity conducting member 61a is two times or more the thickness D of the plate-shaped particular electricity conducting member 61a. In the present embodiment, the thickness D is 80 µm, and the width W is 250 µm. Also, the height H of the bent portion 61w is two times or more the thickness D. In the present embodiment, the height H is 300 µm (0.3 mm). Notably, the remaining particular electricity conducting members 61b, 61c, and 61d have the same thickness D and width W as the particular electricity conducting member 61a, and each have a single bent portion having the height H.

Thus, even when the particular electricity conducting members 61a to 61d are heated as a result of the heating by the heater section 42 and are then cooled; i.e., the particular electricity conducting members 61a to 61d receive thermal stresses, their bent portions 61w absorb thermal contractions of the particular electricity conducting members 61a to 61d. As a result, it is possible to prevent breakage of the particular electricity conducting members 61a to 61d and prevent the energization pads 42p joined to the particular electricity conducting members 61a to 61d from being pulled and coming off. Even when the gas sensor 1 falls and receives an impact, deformation of the particular electricity conducting members 61a to 61d is suppressed, because the particular electricity conducting members 61a to 61d have a plate-like shape and are therefore high in rigidity, and each of the particular electricity conducting members 61a to 61d has the bent portion 61w bent in the thickness direction T and can effectively relieve the impact force generated as a result of the falling. Therefore, even when the gas sensor 1 receives an impact as a result of falling, a deterioration in the gas detection accuracy is suppressed. Specially, even when the gas sensor 1 receives an impact as a result of falling, a positional shift of the sensor element 40 suspended at a particular position within the opening 50h of the wiring board 50 is less likely to occur, and the volume of the gas atmospheric space around the sensor element 40 (the detection section 43) within the case 10 is restrained from changing as a result of falling of the gas sensor 1. The positional shift of the sensor element 40 and the changing of the gas atmospheric space would otherwise occur as a result of deformation of the particular electricity conducting members 61a to 61d. Thus, a deterioration in the gas detection accuracy is suppressed.

Also, in the above-described embodiment, the bent portion 61w has an arch-like shape. Since this shape allows the bent portion 61w to smoothly expand and contract, thermal contraction of the particular electricity conducting members 61a to 61d can be absorbed more stably. Further, in the above-described embodiment, since the particular electricity conducting members 61a to 61d each have a single bent portion 61w, the structure of the particular electricity conducting members 61a to 61d is simple, which allows provision of the gas sensor 1 which is inexpensive and compact. Also, since each of the bent portions 61w of the particular electricity conducting members 61a to 61d bent unidirectionally in the thickness direction T has a height H two times or more the thickness D, the thermal contractions of the particular electricity conducting members 61a to 61d can be absorbed without fail, and the impact force generated as a result of falling of the gas sensor 1 can be relieved without fail.

Also, as shown in FIG. 2, in the above-described embodiment, the particular electricity conducting members 61a and 61b are joined to the first side 40a of the sensor element 40, and the particular electricity conducting members 61c and 61d are joined to the second side 40b opposite the first side 40a. Since the particular electricity conducting members 61a to 61d fixedly suspend the opposite sides of the sensor element 40, the sensor element 40 can be fixed more reliably. In particular, in the present embodiment, since the particular electricity conducting members 61a to 61d fixedly suspend the sensor element 40 at positions near the four corners of the sensor element 40, the sensor element 40 can be fixed more firmly.

Also, in the above-described embodiment, the main component of the material used for forming the particular electricity conducting members 61a to 61d and the main component of the material used for forming the energization pads 42p and 44p are the same (Pt). Therefore, adhesion between the particular electricity conducting members 61a to 61d and the energization pad 42p and 44pt is improved, and the sensor element 40 can be fixed more reliably.

Needless to say, the present invention is not limited to the above-described embodiment and encompasses various modifications and equivalents within the spirit and scope of the present invention.

The shape and number of the particular electricity conducting members, the shape and number of the bent portions, the shape of the sensor element, etc. are not limited to those in the above-described embodiment. The structures and types of the heater section and the detection section, etc. are not limited to those in the above-described embodiment. Also, in the above-described embodiment, electric resistance welding is employed for joining the particular electricity conducting members to the sensor element and the wiring board; however, the manner of joining is not limited thereto.

### DESCRIPTION OF SYMBOLS

1 gas sensor
40 sensor element
40a first side
40b second side
42 heater section
42p, 44p energization pad
43 detection section
50 wiring board
50h opening
61, 63 electricity conducting member
61 particular electricity conducting member
61a1 first end portion
61a2 second end portion
61w bent portion
G particular gas
T thickness direction of particular electricity conducting member
D thickness of particular electricity conducting member

## Claims

1. A gas sensor comprising:
a wiring board having an opening;
a sensor element disposed within the opening of the wiring board and including a detection section for detecting the concentration of a particular gas and a heater section for heating the detection section; and
a plurality of electricity conducting members each of which has a first end portion joined to the wiring board and a second end portion joined to the sensor element and which establish electrical connection between the wiring board and the sensor element,
the plurality of electricity conducting members fixedly suspending the sensor element within the opening of the wiring board,
wherein at least one of the electricity conducting members is a particular electricity conducting member having a plate-like shape, and the particular electricity conducting member includes at least one bent portion between the second end portion joined to the sensor element and the first end portion joined to the wiring board, the bent portion being bent in a thickness direction of the particular electricity conducting member.

2. A gas sensor according to claim 1, wherein the bent portion is bent into an arch shape.

3. A gas sensor according to claim 1 or 2, wherein the particular electricity conducting member has, as a single bent portion, the bent portion bent unidirectionally in the thickness direction of the particular electricity conducting member, and the bent portion has a height two times or more a thickness of the particular electricity conducting member.

4. A gas sensor according to any one of claim 1 to 3, wherein
the number of the particular electricity conducting members is at least two,
the sensor element has a quadrilateral shape in plan view,
one of the particular electricity conducting members is joined to the sensor element and the wiring board in such a manner as to extend across a first side of the sensor element which is one of four sides of the sensor element defining a peripheral edge thereof, and
the other of the particular electricity conducting members is joined to the sensor element and the wiring board in such a manner as to extend across a second side of the sensor element which is another of the four sides and is located opposite the first side.

5. A gas sensor according to any one of claim 1 to 4, wherein an electrically conductive energization pad which is welded to the particular electricity conducting member is provided on a surface of the sensor element, and a main component of a material used to form the energization pad is the same as a main component of a material used to form the particular electricity conducting member.
